# EUROPEAN PATENT APPLICATION

(11) **EP 4 410 341 A1**
(43) Date of publication of application: **07.08.2024**
(21) Application number: 21958012.3
(22) Date of filing: 27.09.2021
(51) Int. Cl.: A61M 16/00

(54) **MEDICAL VENTILATION DEVICE AND MEDICAL DEVICE SYSTEM**

(71) Applicant: Shenzhen Mindray Bio-Medical Electronics Co., Ltd, Shenzhen, Guangdong 518057 (CN)
(72) Inventor: HUANG, Jiping, Shenzhen, Guangdong 518057 (CN); PAN, Ruiling, Shenzhen, Guangdong 518057 (CN); TIAN, Wentao, Shenzhen, Guangdong 518057 (CN)
(74) Representative: KIPA AB
(86) International application number: PCT/CN2021/120896
(87) International publication number: WO 2023/044894

(57) **Abstract**

A medical ventilation device (110) and a medical device system. The medical ventilation device (110) comprises a patient line (113), a pressure generation apparatus (112), a controller (111), a physical connection structure (114), and a communication interface (115). The physical connection structure (114) is used to detachably mount a first monitor (130) on a main body of the medical ventilation device (110). The communication interface (115) performs information communication with the first monitor (130) when the first monitor (130) is mounted on the medical ventilation device (110). The first monitor (130) is used to monitor the patient during first treatment, and acquire operating data of a primary medical ventilation device (120). The primary medical ventilation device (120) is configured to perform ventilation therapy on the patient on the basis of a configuration adapted to the patient during a first treatment period. The first monitor (130) is also used to monitor the patient during a second treatment period. The controller (111) is also used to obtain operating data of the primary medical ventilation device (120) from the first monitor (130) by means of the communication interface (115), and control the pressure generation apparatus (112) to perform a ventilation treatment on the patient on the basis of the working data during the second treatment period.

## Description

### TECHNICAL FIELD

This disclosure relates to a technical field of medical device, and more specifically to a medical ventilation device and a medical device system.

### BACKGROUND

Patients using mechanical ventilation in clinical situations often have scenarios which need to swich ventilators. For example, a patient using a bedside ventilator in ICU need to go out for CT, B-ultrasound, and other examinations. At this time, the patient needs to switch from an intensive care ventilator to a dedicated portable ventilator. Another example is that a ventilator currently used by the patient has problems or malfunctions, so the patient needs to switch to another ventilator. Switching between ventilators requires medical staff to reset configuration data and/or ventilation monitoring data, and other working data of ventilator, which is complicated and increases workload of doctor. If a treatment plan is adjusted when replacing a ventilator, it may have adverse effects on the patient. For example, some data show that "adjustment of treatment plan before transport increases a probability of adverse event(s)."

Some existing data transfer solutions, such as use(s) of USB and other storage devices or dedicated respiratory pipes, can realize configuration transfer between two ventilators. There is no need for medical staff to repeatedly set configuration of the ventilator, and the ventilation treatment plans for patient before and after switching can be guaranteed to be consistent, which ensures a continuity of ventilation treatment. However, these data transfer solutions require additional data transfer steps, which complicates the transfer process, and the device introduced for data transfer has shortcomings, such as easy loss, high cost, and vulnerability to damage.

Therefore, it is necessary to propose a new medical ventilation device and medical device system to solve above problem(s).

### SUMMARY

A series of simplified concepts are introduced into summary of this disclosure, and are further elaborated in specific embodiments of this disclosure. The summary of this disclosure does not attempt to define key and necessary technical characteristics of the claimed technical solution, nor attempt to determine a protection scope of the claimed technical solution.

A first aspect according to an embodiment of this disclosure provides a medical ventilation device, including: a patient pipeline, which is connected with a patient interface, so as to deliver a respiratory gas to an airway of a patient; a pressure generation apparatus, which is configured to generate the respiratory gas at a preset pressure and deliver said respiratory gas to the patient; a controller, which is configured to control the pressure generation apparatus to perform a ventilation treatment on the patient; wherein, the medical ventilation device is arranged with a physical connection structure, which is configured to assemble a first monitor with a main body of the medical ventilation device, which assembly is capable of being disassembled; wherein the medical ventilation device further includes a communication interface which is configured to perform information communication with the first monitor, when the first monitor is assembled with the medical ventilation device; the first monitor is configured to monitor the patient during a first treatment period and obtain working data of a primary medical ventilation device; wherein the primary medical ventilation device is configured to perform a ventilation treatment on the patient based on configuration(s) which is(are) adapted to the patient during the first treatment period; the first monitor is further configured to monitor the patient during a second treatment period; the controller is further configured to obtain, from the first monitor through the communication interface, the working data of the primary medical ventilation device, and control the pressure generation apparatus to perform a ventilation treatment on the patient based on the working data during the second treatment period.

A second aspect according to an embodiment of this disclosure provides a medical ventilation device, including: a patient pipeline, which is connected with a patient interface, so as to deliver a respiratory gas to an airway of a patient; a pressure generation apparatus, which is configured to generate the respiratory gas at a preset pressure and deliver said respiratory gas to the patient; a controller, which is configured to control the pressure generation apparatus to perform a ventilation treatment on the patient; wherein the medical ventilation device further includes a communication interface, which is configured to establish communicative connection with a forwarding device through a network communication link; wherein the forwarding device is configured to obtain working data of a primary medical ventilation device during a first treatment period; wherein the primary medical ventilation device is configured to perform a ventilation treatment on the patient based on configuration(s) which is(are) adapted to the patient during the first treatment period; the controller is further configured to obtain, from the forwarding device through the network communication link, the working data of the primary medical ventilation device, and control the pressure generation apparatus to perform a ventilation treatment on the patient based on the working data during a second treatment period.

A third aspect according to an embodiment of this disclosure provides a medical ventilation device, including: a patient pipeline, which is connected with a patient interface, so as to deliver a respiratory gas to an airway of a patient; a pressure generation apparatus, which is configured to generate the respiratory gas at a preset pressure and deliver said respiratory gas to the patient; and a controller, which is configured to control the pressure generation apparatus to perform a ventilation treatment on the patient; wherein the medical ventilation device further includes a communication interface, which is configured to establish a network communication link with a primary medical ventilation device for information communication; the primary medical ventilation device is configured to perform a ventilation treatment on the patient based on configuration(s) which is(are) adapted to the patient during a first treatment period; the controller is further configured to obtain, through the communication interface, working data of the primary medical ventilation device, and control the pressure generation apparatus to perform a ventilation treatment on the patient based on the working data during a second treatment period.

A fourth aspect according to an embodiment of this disclosure provides a medical device system, including a medical ventilation device and a first monitor; wherein the medical ventilation device, includes: a patient pipeline, which is connected with a patient interface, so as to deliver a respiratory gas to an airway of a patient; a pressure generation apparatus, which is configured to generate the respiratory gas at a preset pressure and deliver said respiratory gas to the patient; and a controller, which is configured to control the pressure generation apparatus to perform a ventilation treatment on the patient; wherein, the medical ventilation device is arranged with a physical connection structure, which is configured to assemble a first monitor with a main body of the medical ventilation device, which assembly is capable of being disassembled; wherein the medical ventilation device further includes a communication interface which is configured to perform information communication with the first monitor, when the first monitor is assembled with the medical ventilation device; the first monitor is configured to monitor the patient during a first treatment period and obtain working data of a primary medical ventilation device; wherein the primary medical ventilation device is configured to perform a ventilation treatment on the patient based on configuration(s) which is(are) adapted to the patient during the first treatment period; the first monitor is further configured to monitor the patient during a second treatment period; the controller is further configured to obtain, from the first monitor through the communication interface, the working data of the primary medical ventilation device, and control the pressure generation apparatus to perform a ventilation treatment on the patient based on the working data during the second treatment period.

A fifth aspect according to an embodiment of this disclosure provides a medical device system, including a medical ventilation device, a primary medical ventilation device and an intermediate medical device; the primary medical ventilation device is configured to perform a ventilation treatment on a patient based on configuration(s) which is(are) adapted to the patient during a first treatment period; the intermediate medical device is configured to execute designated software program(s), so as to perform corresponding medical function(s); the intermediate medical device is further configured to obtain working data of the primary medical ventilation device during the first treatment period; the medical ventilation device is configured to obtain, from the intermediate medical device, the working data of the primary medical ventilation device during the first treatment period, and to perform a ventilation treatment on the patient based on the working data of the primary medical ventilation device during a second treatment period; wherein the medical ventilation device includes: a patient pipeline, which is connected with a patient interface, so as to deliver a respiratory gas to an airway of the patient; a pressure generation apparatus, which is configured to generate the respiratory gas at a preset pressure and deliver said respiratory gas to the patient; and a controller, which is configured to control the pressure generation apparatus to perform a ventilation treatment on the patient.

The medical ventilation device and medical device system according to embodiments of this disclosure enable a transferring process of working data from a primary medical ventilation device to a medical ventilation device convenient and smooth.

### BRIEF DESCRIPTION OF THE DRAWINGS

In order to provide a clearer explanation of technical solutions in embodiments of this disclosure, a brief introduction is given to the accompanying drawings required in the description of the embodiments. It is evident that the accompanying drawings in the following description are only some embodiments of this disclosure. For those skilled in the art, other accompanying drawings can be obtained based on these drawings without any creative effort.
FIG. 1 is a structural diagram of a medical ventilation device according to an embodiment of this disclosure.
FIG. 2 is a structural diagram of a medical ventilation device based on another embodiment of this disclosure.
FIG. 3 is a structural diagram of a medical ventilation device based on a further embodiment of this disclosure.

### DESCRIPTION OF THE PREFERRED EMBODIMENTS

In the following description, a large number of specific details are provided to provide a more thorough understanding of this disclosure. However, it is evident to those skilled in the art that this disclosure can be implemented without the need for one or more of these details. In other examples, in order to avoid confusion with this disclosure, some well-known technical characteristics in this field are not described.

It should be understood that this disclosure can be implemented in different forms and should not be limited to the embodiments proposed here. On the contrary, providing these embodiments make the disclosure thorough and complete, and fully convey the scope of this disclosure to those skilled in the art. In the drawings, size(s) and relative size(s) of layers and regions may be exaggerated for clarity. The same reference numbers refer to the same elements throughout.

The purpose of the terminology used here is only to describe specific embodiments and is not a limitation of this disclosure. When used here, the singular forms of "an", "a", and "the/said" are also intended to include the plural form, unless the context clearly indicates another way. It should also be understood that the terms, such as "including", "and/or", "comprising", when used in this disclosure, determine the presence of the characteristics, integers, steps, operations, components, and/or elements, but do not exclude the presence or addition of one or more other characteristics, integers, steps, operations, components, and/or elements. When used here, the term "and/or" includes any and all combinations of related listed items.

In order to fully understand this disclosure, a detailed structure is provided in the following description to explain the technical solution proposed in this disclosure. The optional embodiments of this disclosure are described in detail below, however, in addition to these detailed description, this disclosure may also have other embodiments.

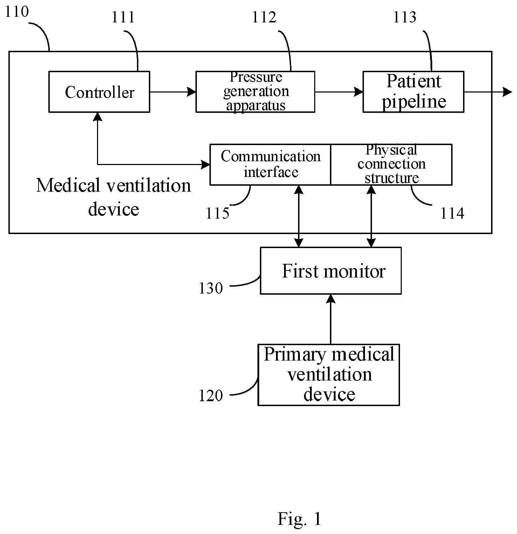

FIG. 1 is a structural diagram of a medical ventilation device according to an embodiment of this disclosure. As shown in FIG. 1, a medical ventilation device 110 includes a controller 111, a pressure generation apparatus 112, a patient pipeline 113, a physical connection structure 114 and a communication interface 115.

The patient pipeline 113 is connected with a patient interface, so as to deliver a respiratory gas to an airway of a patient.

The pressure generation apparatus 112 is configured to generate the respiratory gas at a preset pressure and deliver said respiratory gas to the patient.

The controller 111 is configured to control the pressure generation apparatus 112 to perform a ventilation treatment on the patient.

The medical ventilation device 110 is arranged with a physical connection structure 114, wherein the physical connection structure 114 is configured to assemble a first monitor 130 with a main body of the medical ventilation device, which assembly is capable of being disassembled. The medical ventilation device 110 further includes the communication interface 115, which is configured to perform information communication with the first monitor 130, when the first monitor 130 is assembled with the medical ventilation device 110.

The first monitor 130 is configured to monitor the patient during a first treatment period and obtain working data of a primary medical ventilation device 120; wherein the primary medical ventilation device 120 is configured to perform a ventilation treatment on the patient based on configuration(s) which is(are) adapted to the patient during the first treatment period.

The first monitor 130 is further configured to monitor the patient during a second treatment; and the controller 110 is further configured to obtain, from the first monitor 130 through the communication interface 115, the working data of the primary medical ventilation device 120, and control the pressure generation apparatus 112 to perform a ventilation treatment on the patient based on the working data during the second treatment period.

Illustratively, the medical ventilation device 110 includes a portable ventilator, and the primary medical ventilation device 120 includes an intensive care ventilator. A ventilator is an artificial mechanical ventilation device, which is configured to control or assist a respiratory movement of patient, so as to achieve gas exchange in lungs and reduce respiratory work of patient, thus facilitating a recovery of respiratory function.

The controller 111 can be used as a controlling and computing centre of the medical ventilation device 110, and can implement one or more functions of data acquisition control, data processing, data storage, data output control, display control, and so on, through computer executable programs or hardware circuits.

The pressure generation apparatus 112 is connected with the patient interface through a patient pipeline 113. The patient interface may include a mask, a nasal mask, a nasal cannula, an endotracheal tube, etc., which is attached to the patient and capable of delivering to the patient, the respiratory gas which is generated by the pressure generation apparatus 112.

Illustratively, working data of the medical ventilation device 110 and working data of the primary medical ventilation device 120 includes configuration data and/or ventilation monitoring data.

The configuration data include one or more of: a ventilation mode configuration, a ventilation parameter configuration, and an alarm limit configuration. Wherein, a ventilation mode includes at least one of assist/control ventilation (A/C), intermittent positive pressure ventilation (IPPV), intermittent mandatory ventilation (IMV), synchronized intermittent mandatory ventilation (SIMV), continuous positive airway pressure ventilation (CPAP), positive end-expiratory pressure ventilation (PEEP), deep inhalation (SIGH), etc. A ventilation parameter includes at least one of a tidal volume, a respiratory frequency, an inspiratory time, an inspiratory flow rate, a PEEP value, an oxygen concentration, etc. An alarm limit includes at least one of: upper and lower limits of an airway pressure, upper and lower limits of ventilation in each minute, upper and lower limits of tidal volume, upper and lower limits of inhaled oxygen concentration, an apnoea alarm time, etc.

The ventilation monitoring data may include one or more of a flow rate of ventilation gas, an airway pressure, a respiratory frequency, a tidal volume, an inspiratory time, and compliance of respiratory system or lungs. It should be noted that the ventilation monitoring data can be directly monitored, or can be calculated after detecting certain basic parameters.

Illustratively, the first monitor 130 includes a transport monitor for monitoring the patient during a first treatment period and a second treatment period. A monitor is configured to monitor physiological parameter(s) of the patient. The monitor includes sensor(s) for measuring one or more physiological parameters, such as, electrocardiogram (ECG), respiration, blood oxygen, blood pressure, body temperature, etc. In one embodiment, a sensor for measuring physiological parameter(s) at least includes at least one of a sensor for measuring an electrocardiogram signal parameter, a sensor for measuring a respiratory parameter, a sensor for measuring a body temperature parameter, a sensor for measuring a blood oxygen parameter, a sensor for measuring a non-invasive blood pressure parameter, a sensor for measuring an invasive blood pressure parameter, etc. Each sensor for measuring physiological parameter(s) is connected with an externally inserted sensor accessory through a corresponding sensor interface. A sensor accessor includes a detection accessory for detecting physiological parameter(s), such as ECG, respiration, blood oxygen, blood pressure, and body temperature.

Exemplarily, the first treatment period is a bedside treatment period, and the second treatment period is a transport treatment period.

In one embodiment, the patient is mechanically ventilated by the primary medical ventilation device 120 during a first treatment period, and the patient is mechanically ventilated by the medical ventilation device 110 during a second treatment period. Further, during the first treatment period, the medical staff adjusts configuration(s) of the primary medical ventilation device 120 based on a condition of the patient, so that the primary medical ventilation device 120 performs a ventilation treatment on the patient based on configuration(s) which is(are) adapted to the patient.

In one embodiment, during a first treatment period, the first monitor 130 monitors the patient and obtains working data of the primary medical ventilation device 120, wherein the working data include configuration data and/or ventilation monitoring data of the primary medical ventilation device, when the primary medical ventilation device performs the ventilation treatment on the patient during the first treatment period.

In one embodiment, the first monitor 130 is in communicative connection with the primary medical ventilation device 120, wherein the communicative connection includes a wired communicative connection or wireless communicative connection, as described below.

In one embodiment, the first monitor 130 is in communicative connection with the primary medical ventilation device 120 through a wired interface, such as an Ethernet port.

In one embodiment, the first monitor 130 and the primary medical ventilation device 120 include hot-plug connection. The primary medical ventilation device 120 is arranged with a plug-in slot which supports hot-plugging of the first monitor 130. The first monitor 130 synchronizes with the primary medical ventilation device 120 through a communication interface of the plug-in slot for obtaining the working data of the primary medical ventilation device 120.

In one embodiment, the first monitor 130 is in communicative connection with the primary medical ventilation device 120 through a wireless interface, and the wireless interface includes at least one of a near-field communication interface, a cellular network, and Wi-Fi. The near-field communication interface includes at least one of an infrared interface, a Bluetooth interface, or any other suitable wireless interface. The cellular network includes at least one: a GSM network, a GPRS network, an LTE network, a 2G network, a 3G network, a 4G network, a 5G network or a future communication network.

In one embodiment, pairing connection between the first monitor 130 and the primary medical ventilation device 120 includes short-range wireless communicative connection. Specifically, the primary medical ventilation device 120 provides a hotspot, and the first monitor 130 pairs with the primary medical ventilation device 120 based on identification information of the primary medical ventilation device 120, and connects with a wireless hotspot provided by the paired primary medical ventilation device 120, so as to establish connection with the paired primary medical ventilation device 120. The primary medical ventilation device 120 transmits configuration data of the primary medical ventilation device 120 to the paired first monitor 130. Further, a display interface of the primary medical ventilation device 120 can be arranged to include a parameter display area for displaying medical data, and a navigation area for displaying function option(s). The user can pair and connect the first monitor 130 with the primary medical ventilation device 120 through the navigation area. It should be understood that although the primary medical ventilation device 120 is shown as providing a wireless hotspot, it is not so limited, and the first monitor 130 may also serve as a device that provides a wireless hotspot.

In one embodiment, during a second treatment period, the first monitor 130 is assembled with a main body of the medical ventilation device 110 through a physical connection structure 114, which assembly is capable of being disassembled, wherein the physical connection structure 114 is arranged at the medical ventilation device 110 . When the first monitor 130 is assembled with the medical ventilation device 110, information communication is performed with the first monitor 130 through the communication interface 115, which is arranged at the medical ventilation device 110 .

In one embodiment, the physical connection structure 114 includes a hot-plug structure, the medical ventilation device 110 is arranged with a plug-in slot which supports hot-plugging of the first monitor 130, and the communication interface 115 is arranged at the plug-in slot, wherein the communication interface 115 includes at least one of a contact communication interface, an optical communication interface, a Bluetooth communication interface, and a WIFI communication interface.

In one embodiment, the medical ventilation device 110 further includes a power supply apparatus and a common power interface. When the first monitor 130 is assembled with the main body of the medical ventilation device 110, the power supply apparatus is configured to supply power for the medical ventilation device 110, and to supply power for the first monitor 130 through the common power interface.

In one embodiment, during a second treatment period, the controller 111 is further configured to control the pressure generation apparatus 112 to perform a ventilation treatment on the patient based on the configuration data and/or the ventilation monitoring data.

In one embodiment, during a first treatment period, the patient is mechanically ventilated by the primary medical ventilation device 120 and monitored by the first monitor 130; while during a second treatment period, the patient is mechanically ventilated by the medical ventilation device 110 and monitored by the first monitor 130. Based on communicative connection between the first monitor 130 and the primary medical ventilation device 120, the working data of the primary medical ventilation device 120 can be obtained. The working data include configuration data and/or ventilation monitoring data of the primary medical ventilation device 120 when performing the ventilation treatment on the patient during the first treatment period. Based on physical connection and communication interface connection between a second monitor and the medical ventilation device 110, the working data of the primary medical ventilation device 120 can be transmitted to the medical ventilation device 110. During the second treatment period, the medical ventilation device 110 controls the pressure generation apparatus to perform a ventilation treatment on the patient based on the working data.

When the primary medical ventilation device 120, the first monitor 130 and the medical ventilation device 110 adopt same data format(s) and protocol(s), for example, when the primary medical ventilation device 120, the first monitor 130 and the medical ventilation device 110 are products of a same series from a same manufacturer, direct transmission for the working data of the primary medical ventilation device 120 between the primary medical ventilation device 120 and the first monitor 130, and between the first monitor 130 and the medical ventilation device 110, can be implemented. The working data of the primary medical ventilation device 120 is received from the primary medical ventilation device 120 and then transmitted to the medical ventilation device 110 through the first monitor 130, and the medical ventilation device 110 performs mechanical ventilation on the patient based on the working data of the primary medical ventilation device 120, so that working data of the medical ventilation device 110 adapts to the patient.

Of course, if the primary medical ventilation device 120, the first monitor 130 and the medical ventilation device 110 adopt different data formats and protocols, the data formats and protocols can also be converted first when transmitting between the devices, so as to satisfy data exchange between devices from different manufacturers.

In one embodiment, the medical ventilation device 110 further includes one or more processors, one or more memories, and the likes, which are interconnected by a bus system and/or other form(s) of connection mechanism(s).

The memory is configured to store various data and executable program(s) generated during related ventilation processes, such as, system program(s) for ventilation device, various application programs or algorithms that implement various specific functions, maintenance data, and maintenance instructions, etc. The memory may include one or more computer program products, which may include various forms of computer-readable storage media, such as volatile memory and/or non-volatile memory. The volatile memory may include, for example, random access memory (RAM) and/or cache memory (cache). The non-volatile memory may include, for example, read-only memory (ROM), hard disk, flash memory, etc. During a monitoring process for objects connected with the ventilation device, if there is any data that needs to be stored locally, such data can be stored in the memory.

The processor may be a central processing unit (CPU), a graphic processing unit (GPU), an application specific integrated circuit (ASIC), a field programmable gate array (FPGA), or other form(s) of processing unit(s) with data processing capability and/or instruction execution capability, and can control other components in the ventilation device to perform desired functions. For example, a processor can include an embedded processor, a processor core, a microprocessor, logic circuit(s), a hardware finite state machine (FSM), a digital signal processor (DSP), a graphic processing unit (GPU), or combination(s) thereof.

In one embodiment, the medical ventilation device 110 further includes an input apparatus, which may be an apparatus used by a user to input instruction(s), and may include one or more of a keyboard, a trackball, a mouse, a microphone, a touch screen, etc., or an input apparatus which is formed by other control buttons. For example, the user can input a first control instruction, a second control instruction, etc., through the input apparatus.

In one embodiment, the medical ventilation device 110 further includes a display apparatus that can output various information (e.g., images or sounds) to outside (e.g., a user), and may include one or more of a display, a speaker, and the likes. When the ventilation device is being maintained, the display apparatus displays relevant information that the ventilation device is being maintained.

In one embodiment, the medical ventilation device 110 may also include a user interface through which a user of the medical ventilation device 110 may control an operation of the medical ventilation device. The user interface may include a display, which may include a touch screen that allows the user to input operation instruction(s) to the medical ventilation device 110 through the display, and/or include one or more control panels, etc., through which the user may control an operation of a monitoring system.

A complete medical ventilation device 110 may also include other components and structures, which are not described again here.

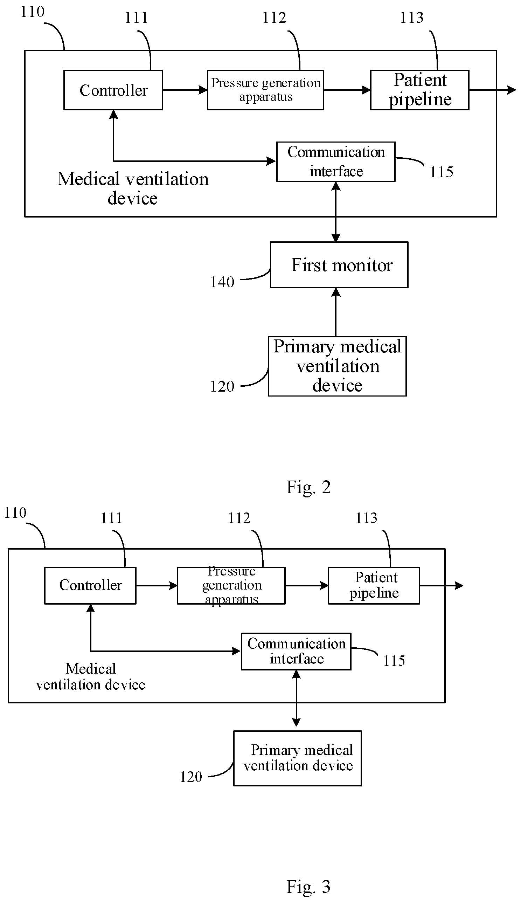

FIG. 2 is a schematic structural diagram of a medical ventilation device according to an embodiment of this disclosure. As shown in FIG. 2, a medical ventilation device 110 includes a controller 111, a pressure generation apparatus 112, a patient pipeline 113, and a communication interface 115.

The patient pipeline 113 is connected with a patient interface, so as to deliver a respiratory gas to an airway of a patient.

The pressure generation apparatus 112 is configured to generate the respiratory gas at a preset pressure and deliver said respiratory gas to the patient.

The controller 111 is configured to control the pressure generation apparatus 112 to perform a ventilation treatment on the patient.

The medical ventilation device further includes a communication interface 115, which is configured to establish communicative connection with a forwarding device 140 through a network communication link.

The forwarding device 140 is configured to obtain working data of a primary medical ventilation device 120 during a first treatment period; wherein the primary medical ventilation device 120 is configured to perform a ventilation treatment on the patient based on configuration(s) which is(are) adapted to the patient during the first treatment period.

The controller 111 is further configured to obtain the working data of the primary medical ventilation device 120 from the forwarding device 140 through the network communication link, and control the pressure generation apparatus to perform a ventilation treatment on the patient based on the working data during a second treatment period.

In one embodiment, the network communication link includes a wired communication mode and/or a wireless communication mode. In this embodiment, the medical ventilation device 110 and the forwarding device 140 transmit data through a network communication link, which is different from a manual copy and transfer of data through using a direct storage device, such as a USB flash drive. It is more convenient, reduces device management, and is easier to realize remote transmission.

In one embodiment, the medical ventilation device 110 includes a portable ventilator, the primary medical ventilation device 120 includes an intensive care ventilator, the first treatment period is a bedside treatment period, and the second treatment period is a transport treatment period. The patient is mechanically ventilated by the primary medical ventilation device 120 during the first treatment period, and the patient is mechanically ventilated by the medical ventilation device 110 during the second treatment period. Further, during the first treatment period, the medical staff adjusts configuration(s) of the primary medical ventilation device 120 based on a condition of the patient, so that the primary medical ventilation device 120 performs a ventilation treatment on the patient based on configuration(s) which is(are) adapted to the patient.

In one embodiment, the forwarding device 140 includes a first monitor and a second monitor. During a first treatment period, the first monitor and the second monitor both monitor the patient. During a second treatment period, the first monitor monitors the patient.

In one embodiment, during a first treatment period, the second monitor is in communicative connection with the primary medical ventilation device 120, so as to obtain working data of the primary medical ventilation device 120, the first monitor and the second monitor are in communicative connection, such that the second monitor transmits the obtained working data of the primary medical ventilation device 120 to the first monitor.

In one embodiment, the working data include configuration data and/or ventilation monitoring data of the primary medical ventilation device 120 when performing the ventilation treatment on the patient during the first treatment period. The configuration data include one or more of a ventilation mode configuration, a ventilation parameter configuration, and an alarm limit configuration. The ventilation monitoring data include one or more of a flow rate of ventilation gas, an airway pressure, a respiratory frequency, a tidal volume, an inspiratory time, and compliance of respiratory system or lungs.

In one embodiment, the second monitor is in communicative connection with the primary medical ventilation device 120, wherein the communicative connection includes wired communicative connection or wireless communicative connection. In one embodiment, the first monitor is in communicative connection with the second monitor, wherein the communicative connection includes wired communicative connection or wireless communicative connection. Those skilled in the art can understand connection modes of wired communicative connection or wireless communicative connection based on the above. For the sake of simplicity, the details are not repeated here.

In one embodiment, the first monitor serves as a module of the second monitor, which module is capable of being dissembled. In one embodiment, the second monitor includes a multi-parameter monitor, and the first monitor is a multi-parameter monitoring module of the second monitor. The first monitor can be arranged outside a housing of the second monitor as an independent external parameter module; or can be inserted into a host (including a main control board) of the second monitor to form a plug-in monitor and serve as a part of a second monitor; or can be connected with a host (including a main control board) of the second monitor through a cable to form an external parameter module which serves as an external accessory of the second monitor.

In one embodiment, the second monitor is arranged with a plug-in structure which supports hot-plugging of the first monitor, and the first monitor is in communicative connection with the second monitor through a communication interface in the plug-in structure. In one embodiment, the plug-in slot is a plug-in slot that supports hot-plugging. The plug-in slot matches with an interface of the first monitor, and can not only be a mechanical structure that accommodates the first monitor, but also can have a data transmission interface which implements data exchange with the first monitor, and further can have a power interface for supplying power to the first monitor. The plug-in slot is connected with a main control board of the second monitor, and data of the first monitor can be transmitted to a host of the second monitor through the data transmission interface, and instructions from the host of the second monitor can also be transmitted through the data transmission interface to the first monitor.

In one embodiment, although the first monitor serves as a module of the second monitor, which module is capable of being dissembled, the first monitor at least includes a power supply module and a main control circuit. In one embodiment, the power supply module is configured to control power-on and power-off of an entire machine, a power-on sequence of each power domain within a board card, and battery charging and discharging. The main control circuit includes at least one processor and at least one memory.

In one embodiment, during a second treatment period, the first monitor is configured to monitor the patient, and the first monitor is in communicative connection with the medical ventilation device 110 and is configured to transmit working data of the primary medical ventilation device 120 to the medical ventilation device through a network communication link. In one embodiment, the communicative connection between the first monitor and the medical ventilation device 110 is wired communicative connection or wireless communicative connection.

In one embodiment, the forwarding device 140 includes a first monitor and a second monitor. During a first treatment period, the patient is mechanically ventilated by the primary medical ventilation device 120, and monitored by the first monitor and the second monitor, while during a second treatment period, the patient is mechanically ventilated by the medical ventilation device 110 and monitored by the first monitor.

Based on communicative connection between the second monitor and the primary medical ventilation device 120, working data of the primary medical ventilation device 120 can be obtained. The working data include configuration data and/or ventilation monitoring data of the primary medical ventilation device 120 when performing the ventilation treatment on the patient during the first treatment period. Based on communicative connection between the second monitor and the first monitor, the working data of the primary medical ventilation device 120 is transmitted to the first monitor. Based on communicative connection between the first monitor and the medical ventilation device 110, the working data of the primary medical ventilation device 120 is transmitted to the medical ventilation device 110. During the second treatment period, the medical ventilation device 110 controls the pressure generation apparatus to perform a ventilation treatment on the patient based on the working data.

In one embodiment, the forwarding device 140 includes a second monitor, which is configured to monitor the patient during a first treatment period, wherein the second monitor is further in communicative connection with the primary medical ventilation device 120, so as to obtain working data of the primary medical ventilation device 120. The second monitor is also in communicative connection with the medical ventilation device 110, and is further in communicative connection with the primary medical ventilation device through a network communication link, so as to transmit the working data of the primary medical ventilation device 120 to the medical ventilation device 110.

In one embodiment, the forwarding device 140 includes a second monitor. During a first treatment period, the patient is mechanically ventilated by the primary medical ventilation device 120 and monitored by the second monitor, and during a second treatment, the patient is mechanically ventilated by the medical ventilation device 110. Based on communicative connection between the second monitor and the primary medical ventilation device 120, working data of the primary medical ventilation device 120 can be obtained. The working data include configuration data and/or ventilation monitoring data of the primary medical ventilation device 120 when performing the ventilation treatment on the patient during the first treatment period. Based on communicative connection between the second monitor and the medical ventilation device 110, the working data of the primary medical ventilation device 120 can be transmitted to the medical ventilation device 110 through a network communication link. During the second treatment period, the medical ventilation device 110 controls the pressure generation apparatus to perform a ventilation treatment on the patient based on the working data.

In one embodiment, the forwarding device 140 includes a first monitor. During a first treatment period, the first monitor is configured to monitor the patient, and the first monitor is in communicative connection with the primary medical ventilation device 120, so as to obtain working data of the primary medical ventilation device 120. During a second treatment period, the first monitor is configured to monitor the patient, and the first monitor is in communicative connection with the medical ventilation device 110 and configured to transmit the working data of the primary medical ventilation device 120 to the medical ventilation device 110 through a network communication link.

In one embodiment, the forwarding device 140 includes a first monitor. During a first treatment period, the patient is mechanically ventilated by the primary medical ventilation device 120 and monitored by the first monitor, while during a second treatment period, the patient is mechanically ventilated by the medical ventilation device 110 and monitored by the first monitor. Based on communicative connection between the first monitor and the primary medical ventilation device 120, working data of the primary medical ventilation device 120 can be obtained. The working data include configuration data and/or ventilation monitoring data of the primary medical ventilation device 120 when performing the ventilation treatment on the patient during the first treatment period. Based on communicative connection between the first monitor and the medical ventilation device 110, the working data of the primary medical ventilation device 120 can be transmitted to the medical ventilation device 110 through a network communication link. During the second treatment period, the medical ventilation device 110 controls the pressure generation apparatus to perform a ventilation treatment on the patient based on the working data.

In one embodiment, the forwarding device 140 includes a central information device, which is configured to obtain working data of the primary medical ventilation device 120 and forward said data to the medical ventilation device 110 .

In one embodiment, the central information device includes at least one of a monitoring central station, a ventilator central station, and a hospital information system. The central information device is at least in communicative connection with the primary medical ventilation device 120. The primary medical ventilation device 120 transmits the working data of the primary medical ventilation device 120 to the central information device through an external communication interface. The external communication interface can be one or a combination of local area network interfaces composed of Ethernet, Token Ring, Token Bus, and optical fiber distributed data interface (FDDI) as a backbone of these three networks, may also be one or a combination of wireless interfaces, such as infrared, Bluetooth, WIFI, and WMTS communication, or may also be one or a combination of wired data connection interfaces, such as RS232 and USB. The external communication interface may also be one or a combination of a wireless data transmission interface and a wired data transmission interface.

In one embodiment, the forwarding device 140 includes a central information device. During a first treatment period, the patient is mechanically ventilated by the primary medical ventilation device 120, while during a second treatment period, the patient is mechanically ventilated by the medical ventilation device 110. Based on communicative connection between the central information device and the primary medical ventilation device 120, the central information device can obtain working data of the primary medical ventilation device 120. The working data include configuration data and/or ventilation monitoring data of the primary medical ventilation device 120 when performing the ventilation treatment on the patient during the first treatment period. Based on further communicative connection between the central information device and the medical ventilation device 110, the central information device further forwards the working data of the primary medical ventilation device 120 to the medical ventilation device 110. During the second treatment period, the medical ventilation device 110 controls the pressure generation apparatus to perform a ventilation treatment on the patient based on the working data.

In one embodiment, the forwarding device 140 further includes a second monitor, which is configured to monitor the patient during a first treatment period. The second monitor is further in respective communicative connection with the primary medical ventilation device 120 and the central information device, so as to obtain working data of the primary medical ventilation device 120 and transmit said data to the central information device.

In one embodiment, the forwarding device 140 includes a central information device and a second monitor. During a first treatment period, the patient is mechanically ventilated by the primary medical ventilation device 120 and monitored by the second monitor. During a second treatment period, the patient is mechanically ventilated by the medical ventilation device 110. Based on communicative connection between the second monitor and the primary medical ventilation device 120, working data of the primary medical ventilation device 120 can be obtained. The working data include configuration data and/or ventilation monitoring data of the primary medical ventilation device 120 when performing the ventilation treatment on the patient during the first treatment period. Based on communicative connection between the second monitor and the central information device, the working data of the primary medical ventilation device 120 can be transmitted to the central information device.

In one embodiment, the forwarding device 140 further includes a first monitor, which is configured to monitor the patient during a first treatment period and a second treatment period respectively, wherein the first monitor is further configured to transmit identification information of the patient to the medical ventilation device 110. The medical ventilation device 110 is further configured to obtain working data of the primary medical ventilation device 120, which data corresponds to the identification information, from the central information device based on the identification information.

In one embodiment, the forwarding device 140 includes a central information device and a first monitor. During a first treatment period, the patient is mechanically ventilated by the primary medical ventilation device 120 and monitored by the first monitor. During a second treatment period, the patient is mechanically ventilated by the medical ventilation device 110 and monitored by the first monitor. The first monitor is further configured to transmit identification information of the patient to the medical ventilation device 110. The identification information of the patient includes identity information or location information of the patient, such as ID, clinic number, hospitalization number, department number, support number, etc., of the patient. The medical ventilation device 110 is further configured to obtain the working data of the primary medical ventilation device 120, which data corresponds to the identification information, from the central information device based on the identification information. During the second treatment period, the medical ventilation device 110 controls the pressure generation apparatus to perform a ventilation treatment on the patient based on the working data.

In one embodiment, the forwarding device 140 further includes a first monitor and a second monitor. During a first treatment period, the first monitor and the second monitor are configured to monitor the patient. During a second treatment period, the first monitor is configured to monitor the patient, and the first monitor is further configured to transmit identification information of the patient to the medical ventilation device 110. The medical ventilation device 110 is further configured to obtain working data of the primary medical ventilation device 120 which data corresponds to the identification information, from the central information device based on the identification information.

In one embodiment, the second monitor is arranged with a plug-in structure which supports hot-plugging of the first monitor, and the first monitor is in communicative connection with the second monitor through a communication interface in the plug-in structure. One skilled in the art can understand the connection method between the first monitor and the second monitor based on the foregoing description. For the sake of simplicity, the details are not repeated here.

In one embodiment, the forwarding device 140 includes a central information device, a first monitor and a second monitor. During a first treatment period, the patient is mechanically ventilated by the primary medical ventilation device 120, and monitored by the first monitor and the second monitor. During a second treatment period, the patient is mechanically ventilated by the medical ventilation device 110 and monitored by the first monitor. Based on communicative connection between the second monitor and the primary medical ventilation device 120, working data of the primary medical ventilation device 120 can be obtained. The working data include configuration data and/or ventilation monitoring data of the primary medical ventilation device 120 when performing the ventilation treatment on the patient during the first treatment period. Based on communicative connection between the second monitor and the central information device, the working data of the primary medical ventilation device 120 can be transmitted to the central information device. The first monitor is further configured to transmit identification information of the patient to the medical ventilation device 110. The identification information of the patient includes identity information or location information of the patient, such as ID, clinic number, hospitalization number, department number, support number, etc., of the patient. The medical ventilation device 110 is further configured to obtain the working data of the primary medical ventilation device 120, which data corresponds to the identification information, from the central information device based on the identification information. During the second treatment period, the medical ventilation device 110 controls the pressure generation apparatus to perform a ventilation treatment on the patient based on the working data.

Those skilled in the art can understand other components and structures of the medical ventilation device 110 based on the foregoing description. For simplicity, the details are not described again here.

FIG. 3 is a schematic structural diagram of a medical ventilation device according to an embodiment of this disclosure. As shown in FIG. 3, a medical ventilation device 110 includes a controller 111, a pressure generation apparatus 112, a patient pipeline 113, and a communication interface 115.

The patient pipeline 113 is connected with a patient interface, so as to deliver a respiratory gas to an airway of a patient.

The pressure generation apparatus 112 is configured to generate a respiratory gas at a preset pressure and deliver said respiratory gas to the patient.

The controller 111 is configured to control the pressure generation apparatus 112 to perform a ventilation treatment on the patient.

The medical ventilation device further includes the communication interface 115, which is configured to establish a network communication link with a primary medical ventilation device 120 for information communication. The primary medical ventilation device 120 is configured to perform a ventilation treatment on the patient based on configuration(s) which is(are) adapted to the patient during a first treatment period.

The controller 111 is further configured to obtain, from the primary medical ventilation device 120 through the communication interface 115, working data of the primary medical ventilation device, and control the pressure generation apparatus to perform a ventilation treatment on the patient based on the working data during a second treatment period.

In one embodiment, the medical ventilation device 110 includes a portable ventilator, the primary medical ventilation device 120 includes an intensive care ventilator, the first treatment period is a bedside treatment period, and the second treatment period is a transport treatment period. During the first treatment period, the patient is mechanically ventilated by the primary medical ventilation device 120, while during the second treatment period, the patient is mechanically ventilated by the medical ventilation device 110. Further, during the first treatment period, the medical staff adjusts configuration(s) of the primary medical ventilation device 120 based on a condition of the patient, so that the primary medical ventilation device 120 performs a ventilation treatment on the patient based on configuration(s) which is(are) adapted to the patient.

In one embodiment, the medical ventilation device 110 is further configured to provide a user interaction interface, receive from the user interaction interface a selection instruction which is inputted by a user, and select, based on the selection instruction, a corresponding primary medical ventilation device 120, so as to obtain working data which is transmitted by the primary medical ventilation device 120 based on a corresponding communication mode. Further, during the second treatment period, the medical ventilation device 110 controls the pressure generation apparatus to perform a ventilation treatment on the patient based on the working data.

In one embodiment, the working data include configuration data and/or ventilation monitoring data of the primary medical ventilation device 120 when performing ventilation treatment on the patient during the first treatment period. The configuration data include one or more of a ventilation mode configuration, a ventilation parameter configuration, and an alarm limit configuration. The ventilation monitoring data include one or more of a flow rate of ventilation gas, an airway pressure, a respiratory frequency, a tidal volume, an inspiratory time, and compliance of respiratory system or lungs.

In one embodiment, the network communication link includes a wired communication mode and/or a wireless communication mode. In this embodiment, a communication mode of the network communication link includes one or more of unicast, broadcast, and multicast. The medical ventilation device 110 and the primary medical ventilation device 120 establish the network communication link through a Bluetooth communication mode or a WIFI communication mode. The medical ventilation device is further configured to pair with the primary medical ventilation device through a Bluetooth pairing mode or an NFC pairing mode, so as to establish the network communication link.

In one embodiment, the patient is mechanically ventilated by the primary medical ventilation device 120 during a first treatment period, while the patient is mechanically ventilated by the medical ventilation device 110 during a second treatment period. Based on a network communication link between the medical ventilation device 110 and the primary medical ventilation device 120, the medical ventilation device 110 can obtain the working data of the primary medical ventilation device 120, wherein the working data include configuration data and/or ventilation monitoring data of the primary medical ventilation device 120 when performing the ventilation treatment on the patient during the first treatment period. During the second treatment period, the medical ventilation device 110 controls the pressure generation apparatus to perform a ventilation treatment on the patient based on the working data.

In this embodiment, the medical ventilation device 110 and the primary medical ventilation device 120 perform data transmission through a network communication link, which is different from a manual copy and transfer of data through using a direct storage device, such as a USB flash drive. It is more convenient, reduces device management, and is easier to realize remote transmission.

Those skilled in the art can understand other components and structures of the medical ventilation device 110 based on the foregoing description. For simplicity, the details are not described again here.

This disclosure further provides a medical device system, including a medical ventilation device and a first monitor; wherein the medical ventilation device includes:
a patient pipeline, which is connected with a patient interface, so as to deliver a respiratory gas to an airway of a patient;
a pressure generation apparatus, which is configured to generate the respiratory gas at a preset pressure and deliver said respiratory gas to the patient;
a controller, which is configured to control the pressure generation apparatus to perform a ventilation treatment on the patient;
wherein, the medical ventilation device is arranged with a physical connection structure, which is configured to assemble a first monitor with a main body of the medical ventilation device, which assembly is capable of being disassembled; wherein the medical ventilation device further includes a communication interface which is configured to perform information communication with the first monitor, when the first monitor is assembled with the medical ventilation device;
the first monitor is configured to monitor the patient during a first treatment period and obtain working data of a primary medical ventilation device; wherein the primary medical ventilation device is configured to perform a ventilation treatment on the patient based on configuration(s) which is(are) adapted to the patient during the first treatment period;
the first monitor is further configured to monitor the patient during a second treatment period; the controller is further configured to obtain, from the first monitor through the communication interface, the working data of the primary medical ventilation device, and control the pressure generation apparatus to perform a ventilation treatment on the patient based on the working data during the second treatment period.

Based on the medical device system provided by this disclosure, the primary medical ventilation device and the first monitor are respectively configured to mechanically ventilate the patient and monitor physiological parameter(s) of the patient during the first treatment period, and synchronize configuration data of the primary medical ventilation device during the first treatment period; the medical ventilation device and the first monitor are respectively configured to mechanically ventilate the patient and monitor physiological parameter(s) of the patient during the second treatment period, and synchronize the primary medical ventilation device during the second treatment period, which enables data transfer between the primary medical ventilation device and the medical ventilation device without introducing additional device and operating steps. Moreover, an operation process of data transfer between is simple and convenient.

This disclosure further provides a medical device system, including a medical ventilation device, a primary medical ventilation device and an intermediate medical device; the primary medical ventilation device is configured to perform a ventilation treatment on a patient based on configuration(s) which is(are) adapted to said patient during a first treatment period; the intermediate medical device is configured to execute designated software program(s), so as to perform corresponding medical function(s); the intermediate medical device is further configured to obtain working data of the primary medical ventilation device during the first treatment period; the medical ventilation device is configured to obtain, from the intermediate medical device, the working data of the primary medical ventilation device during the first treatment period, and to perform a ventilation treatment on said patient based on the working data during a second treatment period; wherein the medical ventilation device includes:
a patient pipeline, which is connected with a patient interface, so as to deliver a respiratory gas to an airway of a patient;
a pressure generation apparatus, which is configured to generate the respiratory gas at a preset pressure and deliver said respiratory gas to said patient; and
a controller, which is configured to control the pressure generation apparatus to perform a ventilation treatment on said patient.

In one embodiment, the medical ventilation device, the primary medical ventilation device, and the intermediate medical device are located in a same communication network.

Based on the medical device system provided by this disclosure, the primary medical ventilation device is configured to mechanically ventilate the patient during the first treatment period, the intermediate medical device obtains the working data of the primary medical ventilation device during the first treatment period, and the medical ventilation device obtains, from the intermediate medical device, the working data of the primary medical ventilation device during the first treatment period, which enables data transfer between the primary medical ventilation device and the medical ventilation device without introducing additional device and operating steps. Moreover, an operation process of data transfer between is simple and convenient. Furthermore, the medical ventilation device performs a ventilation treatment on the patient based on the working data during the second treatment period.

In this embodiment, the intermediate medical device executes designated software program(s) to perform corresponding medical functions. For example, when a monitoring central station serves as the intermediate medical device, it can perform central monitoring functions. In this embodiment, an intermediate medical device with specific medical function(s) is used for data forwarding, which is different from a manual copy and transfer of data through using a direct storage device, such as a USB flash drive. It is more convenient, reduces device management, and is easier to realize remote transmission, and is capable of forming information interconnection between medical devices in the hospital.

Although the exemplary embodiments are described here with reference to the accompanying drawings, it should be understood that the aforementioned exemplary embodiments are only illustrative and are not intended to limit the scope of this disclosure. Ordinary technical personnel in this field can make various changes and modifications without deviating from the scope and spirit of this disclosure. All these changes and modifications are intended to be included within the scope of this disclosure as claimed in the attached claims.

Ordinary technical personnel in this field can realize that the units and algorithm steps described in combination with this disclosure can be implemented in electronic hardware, or a combination of computer software and electronic hardware. Whether these functions are executed in hardware or software depends on the specific application and design constraints of the technical solution. Professional technicians may use different methods to implement the described functions for each specific application, but such implementation should not be considered beyond the scope of this disclosure.

In the several embodiments provided in this disclosure, it should be understood that the disclosed devices and methods can be implemented in other ways. For example, the device embodiments described above are only schematic. For example, the division of the units is only a logical functional division, and there may be other division methods in actual implementations. For example, multiple units or components can be combined or integrated into another device, or some characteristics can be ignored or not executed.

The disclosure provided here provides a large number of specific details. However, it can be understood that the embodiments of this disclosure can be practiced without these specific details. In some examples, well-known methods, structures, and techniques are not shown in detail to avoid blurring the understanding of this disclosure.

Similarly, it should be understood that in order to streamline this disclosure and assist in understanding one or more of the various aspects of this disclosure, in the description of exemplary embodiments of this disclosure, the various characteristics of this disclosure are sometimes grouped together into a single embodiment, diagram, or description thereof. However, the method of this disclosure should not be interpreted as reflecting the intention that the claimed protection of this disclosure requires more characteristics than those explicitly recorded in each claim. More precisely, as reflected in the corresponding claims, the inventive point is that the corresponding technical problem can be solved with less than all characteristics of a single disclosed embodiment. Therefore, the claims following the specific implementation method are explicitly incorporated into the specific implementation method, where each claim itself serves as a separate embodiment of this disclosure.

Ordinary technical personnel in this field can understand that, in addition to mutual exclusion between characteristics, any combination can be configured to combine all characteristics disclosed in this disclosure (including accompanying claims, abstract, and drawings), as well as all processes or units of any method or device so disclosed. Unless otherwise explicitly stated, each characteristic disclosed in this disclosure (including accompanying claims, abstract, and accompanying drawings) may be replaced by alternative characteristics that provide the same, equivalent, or similar purpose.

In addition, Ordinary technical personnel in this field can understand that although some embodiments described herein include certain characteristics rather than other characteristics included in other embodiments, the combination of characteristics of different embodiments means that they are within the scope of this disclosure and form different embodiments. For example, in the claims, any one of the claimed embodiments can be used in any combination.

It should be noted that the above embodiments illustrate this disclosure rather than limit it, and ordinary technical personnel in this field can design alternative embodiments without departing from the scope of the attached claims. In the claims, any reference symbol between parentheses should not be constructed as a restriction on the claims. This disclosure can be implemented with the help of hardware consisting of several different components and with the help of appropriately programmed computers. Among the unit claims that list several devices, several of these devices can be specifically embodied through the same hardware item. The use of words first, second, and third does not indicate any order. These words can be interpreted as names.

## Claims

1. A medical ventilation device, **characterized in that**, comprising:
a patient pipeline, which is connected with a patient interface, so as to deliver a respiratory gas to an airway of a patient;
a pressure generation apparatus, which is configured to generate the respiratory gas at a preset pressure and deliver said respiratory gas to the patient; and
a controller, which is configured to control the pressure generation apparatus to perform a ventilation treatment on the patient;
wherein, the medical ventilation device is arranged with a physical connection structure, which is configured to assemble a first monitor with a main body of the medical ventilation device, which assembly is capable of being disassembled; wherein the medical ventilation device further comprises a communication interface, which is configured to perform information communication with the first monitor, when the first monitor is assembled with the medical ventilation device;
the first monitor is configured to monitor the patient during a first treatment period and obtain working data of a primary medical ventilation device; wherein the primary medical ventilation device is configured to perform a ventilation treatment on the patient based on configuration(s) which is(are) adapted to the patient during the first treatment period;
the first monitor is further configured to monitor the patient during a second treatment period; the controller is further configured to obtain, from the first monitor through the communication interface, the working data of the primary medical ventilation device, and control the pressure generation apparatus to perform a ventilation treatment on the patient based on the working data during the second treatment period.

2. The medical ventilation device according to claim 1, **characterized in that**, the physical connection structure comprises a hot-plug structure, the medical ventilation device is further arranged with a plug-in slot, which supports hot-plugging of the first monitor, the communication interface is arranged at the plug-in slot, and the medical ventilation device is further configured to communicate with the first monitor through the communication interface.

3. The medical ventilation device according to claim 1 or claim 2, **characterized in that**, the communication interface comprises at least one of a contact communication interface, an optical communication interface, a Bluetooth communication interface, and a WIFI communication interface.

4. The medical ventilation device according to any one of claims 1-3, **characterized in that**, further comprising a power supply apparatus and a common power interface; wherein, when the first monitor is assembled with the main body of the medical ventilation device, the power supply apparatus is configured to supply power to the medical ventilation device, and to supply power, through the common power interface, to the first monitor.

5. The medical ventilation device according to any one of claims 1-4, **characterized in that**, the first monitor comprises sensor(s) for measuring multiple physiological parameters, wherein the physiological parameters comprise ECG parameter(s).

6. The medical ventilation device according to any one of claims 1-5, **characterized in that**, the medical ventilation device comprises a portable ventilator, the first monitor comprises a transport monitor, and the primary medical ventilation device comprises an intensive care ventilator.

7. The medical ventilation device according to any one of claims 1-6, **characterized in that**, the first treatment period is a bedside treatment period, and the second treatment period is a transport treatment period.

8. The medical ventilation device according to any one of claims 1-7, **characterized in that**, the working data comprise configuration data and/or ventilation monitoring data of the primary medical ventilation device, when performing the ventilation treatment on the patient during the first treatment period;
wherein the controller is further configured to control the pressure generation apparatus to perform a ventilation treatment on the patient based on the configuration data and/or the ventilation monitoring data during the second treatment period.

9. The medical ventilation device according to claim 8, **characterized in that**, the configuration data comprise one or more of a ventilation mode configuration, a ventilation parameter configuration, and an alarm limit configuration;
the ventilation monitoring data comprise an airway pressure and/or a flow rate.

10. A medical ventilation device, **characterized in that**, comprising:
a patient pipeline, which is connected with a patient interface, so as to deliver a respiratory gas to an airway of a patient;
a pressure generation apparatus, which is configured to generate the respiratory gas at a preset pressure and deliver said respiratory gas to the patient; and
a controller, which is configured to control the pressure generation apparatus to perform a ventilation treatment on the patient;
wherein the medical ventilation device further comprises a communication interface, which is configured to establish communicative connection with a forwarding device through a network communication link;
wherein the forwarding device is configured to obtain working data of a primary medical ventilation device during a first treatment period; wherein the primary medical ventilation device is configured to perform a ventilation treatment on the patient based on configuration(s) which is(are) adapted to the patient during the first treatment period;
the controller is further configured to obtain, from the forwarding device through the network communication link, the working data of the primary medical ventilation device, and control the pressure generation apparatus to perform a ventilation treatment on the patient based on the working data during a second treatment period.

11. The medical ventilation device according to claim 10, **characterized in that**, the forwarding device comprises a first monitor and a second monitor;
during the first treatment period, the first monitor and the second monitor are configured to monitor the patient, the first monitor and the second monitor are in communicative connection with each other, the second monitor is further in communicative connection with the primary medical ventilation device, so as to obtain and transmit to the first monitor the working data of the primary medical ventilation device;
during the second treatment period, the first monitor is configured to monitor the patient, the first monitor is further in communicative connection with the medical ventilation device, so as to transmit the working data of the primary medical ventilation device to the medical ventilation device through the network communication link.

12. The medical ventilation device according to claim 11, **characterized in that**, the communicative connection between the first monitor and the second monitor is wired communicative connection or wireless communicative connection, the communicative connection between the first monitor and the medical ventilation device is wired communicative connection or wireless communicative connection.

13. The medical ventilation device according to claim 11, **characterized in that**, the second monitor is arranged with a plug-in structure which supports hot-plugging of the first monitor, the first monitor is configured to communicate with the second monitor through a communication interface in the plug-in structure.

14. The medical ventilation device according to claim 10, **characterized in that**, the forwarding device comprises a second monitor, which is configured to monitor the patient during the first treatment period, wherein the second monitor is in communicative connection with the primary medical ventilation device, so as to obtain the working data of the primary medical ventilation device; the second monitor is further in communicative connection with the medical ventilation device, so as to transmit the working data of the primary medical ventilation device to the medical ventilation device through the network communication link.

15. The medical ventilation device according to claim 10, **characterized in that**, the forwarding device comprises a first monitor;
during the first treatment period, the first monitor is configured to monitor the patient, the first monitor is in communicative connection with the primary medical ventilation device, so as to obtain the working data of the primary medical ventilation device;
during the second treatment period, the first monitor is configured to monitor the patient, the first monitor is in communicative connection with the medical ventilation device, so as to transmit the working data of the primary medical ventilation device to the medical ventilation device through the network communication link.

16. The medical ventilation device according to claim 15, **characterized in that**, the first monitor comprises sensor(s) for measuring multiple physiological parameters, wherein the physiological parameters comprise ECG parameter(s).

17. The medical ventilation device according to claim 10, **characterized in that**, the forwarding device comprises a central information device, which is configured to obtain and transmit to the medical ventilation device the working data of the primary medical ventilation device.

18. The medical ventilation device according to claim 17, **characterized in that**, the forwarding device further comprises a second monitor, which is configured to monitor the patient during the first treatment period, wherein the second monitor is in respective communicative connection with the primary medical ventilation device and the central information device, so as to obtain and transmit to the central information device the working data of the primary medical ventilation device.

19. The medical ventilation device according to claim 17, **characterized in that**, the forwarding device further comprises a first monitor, which is configured to respectively monitor the patient during the first treatment period and the second treatment period, wherein the first monitor is further configured to transmit identification information of the patient to the medical ventilation device; the medical ventilation device is further configured to obtain, from the central information device based on the identification information, working data of the primary medical ventilation device, which data corresponds to the identification information.

20. The medical ventilation device according to claim 17, **characterized in that**, the forwarding device further comprises a first monitor and a second monitor; during the first treatment period, the first monitor and the second monitor are configured to monitor the patient; during the second treatment period, the first monitor is configured to monitor the patient, the first monitor is further configured to transmit identification information of the patient to the medical ventilation device; the medical ventilation device is further configured to obtain, from the central information device based on the identification information, working data of the primary medical ventilation device, which data corresponds to the identification information.

21. The medical ventilation device according to claim 20, **characterized in that**, the second monitor is arranged with a plug-in structure which supports hot-plugging of the first monitor, the first monitor is configured to communicate with the second monitor through a communication interface in the plug-in structure.

22. The medical ventilation device according to claim 20, **characterized in that**, the central information device is in communicative connection with the primary medical ventilation device, so as obtain said working data; or, the second monitor is in respective communicative connection with the primary medical ventilation device and the central information device, so as to obtain and transmit to the central information device said working data.

23. The medical ventilation device according to any one of claims 20-22, **characterized in that**, the central information device comprises at least one of: a monitoring central station, a ventilator central station, and a hospital information system.

24. The medical ventilation device according to any one of claims 10-23, **characterized in that**, the first treatment period is a bedside treatment period, and the second treatment period is a transport treatment period.

25. The medical ventilation device according to any one of claims 10-24, **characterized in that**, the working data comprise configuration data and/or ventilation monitoring data of the primary medical ventilation device, when performing the ventilation treatment on the patient during the first treatment period;
wherein the controller is further configured to control the pressure generation apparatus to perform a ventilation treatment on the patient based on the configuration data and/or the ventilation monitoring data during the second treatment period.

26. The medical ventilation device according to claim 27, **characterized in that**, the configuration data comprise one or more of a ventilation mode configuration, a ventilation parameter configuration, and an alarm limit configuration;
the ventilation monitoring data comprise an airway pressure and/or a flow rate.

27. A medical ventilation device, **characterized in that**, comprising:
a patient pipeline, which is connected with a patient interface, so as to deliver a respiratory gas to an airway of a patient;
a pressure generation apparatus, which is configured to generate the respiratory gas at a preset pressure and deliver said respiratory gas to the patient; and
a controller, which is configured to control the pressure generation apparatus to perform a ventilation treatment on the patient;
wherein the medical ventilation device further comprises a communication interface, which is configured to establish a network communication link with a primary medical ventilation device for information communication; wherein the primary medical ventilation device is configured to perform a ventilation treatment on the patient based on configuration(s) which is(are) adapted to the patient during a first treatment period;
the controller is further configured to obtain, through the communication interface, working data of the primary medical ventilation device, and control the pressure generation apparatus to perform a ventilation treatment on the patient based on the working data during a second treatment period.

28. The medical ventilation device according to claim 27, **characterized in that**, a communication mode of the network communication link comprises unicast, broadcast, and multicast.

29. The medical ventilation device according to claim 27, **characterized in that**, the medical ventilation device is further configured to provide a user interaction interface; receive, through the user interaction interface, a selection instruction which is inputted by a user; and select, based on the selection instruction, a corresponding primary medical ventilation device, so as to obtain working data which is transmitted by said primary medical ventilation device based on a corresponding communication mode.

30. The medical ventilation device according to claim 27, **characterized in that**, the medical ventilation device is further configured to establish the network communication link with the primary medical ventilation device through a Bluetooth communication mode or a WIFI communication mode.

31. The medical ventilation device according to claim 30, **characterized in that**, the medical ventilation device is further configured to pair with the primary medical ventilation device through a Bluetooth pairing mode or an NFC pairing mode, so as to establish the network communication link.

32. The medical ventilation device according to any one of claims 27-31, **characterized in that**, the working data comprise configuration data and/or ventilation monitoring data of the primary medical ventilation device, when performing the ventilation treatment on the patient during the first treatment period;
wherein the controller is further configured to control the pressure generation apparatus to perform a ventilation treatment on the patient based on the configuration data and/or the ventilation monitoring data during the second treatment period.

33. The medical ventilation device according to claim 32, **characterized in that**, the configuration data comprise one or more of: a ventilation mode configuration, a ventilation parameter configuration, and an alarm limit configuration;
the ventilation monitoring data comprise an airway pressure and/or a flow rate.

34. The medical ventilation device according to any one of claims 27-33, **characterized in that**, the first treatment period is a bedside treatment period, and the second treatment period is a transport treatment period.

35. The medical ventilation device according to any one of claims 27-34, **characterized in that**, the primary medical ventilation device comprises an intensive care ventilator, the medical ventilation device comprises a portable ventilator.

36. The medical ventilation device according to any one of claims 27-35, **characterized in that**, the network communication link comprises a wired communication mode and/or a wireless communication mode.

37. A medical device system, **characterized in that**, comprising a medical ventilation device and a first monitor; wherein the medical ventilation device comprises:
a patient pipeline, which is connected with a patient interface, so as to deliver a respiratory gas to an airway of a patient;
a pressure generation apparatus, which is configured to generate the respiratory gas at a preset pressure and deliver said respiratory gas to the patient; and
a controller, which is configured to control the pressure generation apparatus to perform a ventilation treatment on the patient;
wherein, the medical ventilation device is arranged with a physical connection structure, which is configured to assemble the first monitor with a main body of the medical ventilation device, which assembly is capable of being disassembled; wherein the medical ventilation device further comprises a communication interface, which is configured to perform information communication with the first monitor, when the first monitor is assembled with the medical ventilation device;
the first monitor is configured to monitor the patient during a first treatment period and obtain working data of a primary medical ventilation device; wherein the primary medical ventilation device is configured to perform a ventilation treatment on the patient based on configuration(s) which is(are) adapted to the patient during the first treatment period;
the first monitor is further configured to monitor the patient during a second treatment period; the controller is further configured to obtain, from the first monitor through the communication interface, the working data of the primary medical ventilation device, and control the pressure generation apparatus to perform a ventilation treatment on the patient based on the working data during the second treatment period.

38. A medical device system, **characterized in that**, comprising a medical ventilation device, a primary medical ventilation device and an intermediate medical device; wherein the primary medical ventilation device is configured to perform a ventilation treatment on a patient based on configuration(s) which is(are) adapted to the patient during a first treatment period; the intermediate medical device is configured to execute designated software program(s), so as to perform corresponding medical function(s); the intermediate medical device is further configured to obtain working data of the primary medical ventilation device during the first treatment period; the medical ventilation device is configured to obtain, from the intermediate medical device, the working data of the primary medical ventilation device during the first treatment period, and to perform a ventilation treatment on the patient based on the working data during a second treatment period; wherein the medical ventilation device comprises:
a patient pipeline, which is connected with a patient interface, so as to deliver a respiratory gas to an airway of the patient;
a pressure generation apparatus, which is configured to generate the respiratory gas at a preset pressure and deliver said respiratory gas to the patient; and
a controller, which is configured to control the pressure generation apparatus to perform a ventilation treatment on the patient.

39. The medical device system according to claim 38, **characterized in that**, the medical ventilation device, the primary medical ventilation device, and the intermediate medical device are located in a same communication network.
